(19) Europäisches Patentamt
European Patent Office
Office européen des brevets

(11) **EP 4 670 744 A1**

(12) **EUROPÄISCHE PATENTANMELDUNG**

(43) Veröffentlichungstag:
**31.12.2025   Patentblatt 2026/01**

(21) Anmeldenummer: **24185510.5**

(22) Anmeldetag: **28.06.2024**

(51) Internationale Patentklassifikation (IPC):
**A61K 49/10** (2006.01)     **A61K 49/20** (2006.01)
**G01R 33/28** (2006.01)

(52) Gemeinsame Patentklassifikation (CPC):
**A61K 49/20; A61K 49/10;** G01R 33/282;
G01R 33/5601

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH MA MD TN**

(71) Anmelder: **Christian-Albrechts-Universität zu Kiel
-
Körperschaft des öffentlichen Rechts
24118 Kiel (DE)**

(72) Erfinder:
• **Peters, Josh
  24114 Kiel (DE)**

• **Pravdivtsev, Andrey
  24104 Kiel (DE)**
• **Brahms, Arne
  24105 Kiel (DE)**
• **Herges, Rainer
  24119 Kronshagen (DE)**
• **Hövener, Jan-Bernd
  24105 Kiel (DE)**

(74) Vertreter: **Mertzlufft-Paufler, Cornelius et al
Maucher Jenkins
Patent- und Rechtsanwälte
Urachstraße 23
79102 Freiburg im Breisgau (DE)**

(54) **KONTRASTMITTEL FÜR DIE KERNSPINBASIERTE BILDGEBUNG**

(57)   Es wird ein Kontrastmittel für die kernspinbasierte Bildgebung vorgeschlagen, mit einem einen Kernspin aufweisenden, polarisierbaren Agens (Tracer), wobei Tracer-Moleküle durch Polarisation ihrer Kernspins in einen polarisierten Zustand bringbar sind, und mit einem retardierenden Agens (Retarder), wobei eine molekulare Wechselwirkung von Retarder-Molekülen mit den Tracer-Molekülen eine Verlängerung der T1-Relaxationszeit des Tracers zur Folge hat. Ferner werden ein Verfahren zur Herstellung eines hyperpolarisierten Kontrastmittels, ein Retarder zur Verwendung in einem Kontrastmittel für die kernspinbasierte Bildgebung, ein Kontrastmittel zur Anwendung in einem diagnostischen Verfahren in vivo und/oder in vitro, und die Verwendung eines Retarders in einem Kontrastmittel für die kernspinbasierte Bildgebung zur Verlängerung der T1-Relaxationszeit des Tracers vorgeschlagen.

EP 4 670 744 A1

**Beschreibung**

Die Erfindung betrifft ein Kontrastmittel für die kernspinbasierte Bildgebung.

Die Erfindung betrifft weiter ein Verfahren zur Herstellung eines hyperpolarisierten Kontrastmittels.

Die Erfindung betrifft weiter einen Retarder zur Verwendung in einem Kontrastmittel für die kernspinbasierte Bildgebung.

Die Erfindung betrifft weiter eine Verwendung eines erfindungsgemäßen Retarders.

Ein derartiges Kontrastmittel umfasst zumindest ein einen Kernspin aufweisendes, polarisierbares Agens. Dieses Agens besteht aus Molekülen und weist einen Kernspin auf, da wenigstens eine Atomsorte, aus der das Agens aufgebaut ist, einen Kernspin aufweist. Das Agens ist daher durch Ausrichtung zumindest eines Anteils seiner Kernspins polarisierbar. Somit kann das Agens in an sich bekannter Weise Anwendung in der kernspinbasierten Bildgebung, insbesondere der Magnetresonanztomographie (MRT), finden.

Dieses Agens wird in dieser Anmeldung als Tracer bezeichnet. Der Tracer besteht aus Tracer-Molekülen. Es ist möglich, dass der Tracer bei seiner Anwendung am Patienten an dessen Stoffwechsel teilnimmt, und sich beispielsweise in Bereichen erhöhter Stoffwechselaktivität anreichert. Diese Bereiche werden dann bei der kernspinbasierten Bildgebung hervorgehoben. Es ist jedoch nicht notwendig, dass der Tracer am Stoffwechsel des Patienten teilnimmt. Unter Tracer bzw. Tracer-Molekülen im Sinne dieser Anmeldung können daher auch Stoffe bzw. deren Moleküle verstanden werden, die nicht am Stoffwechsel des Patienten teilnehmen. Diese beispielsweise als Nicht-Stoffwechsel-Stoffe bzw. Nicht-Stoffwechsel-Moleküle bezeichenbare Stoffe bzw. Moleküle können beispielsweise bei der Angiographie, der Untersuchung einer Durchblutung des Patienten, der Untersuchung der Funktion der Blut-Hirn-Schranke, pH-Wert-Messungen, Ionen-Mapping oder der Untersuchung von Organfunktionen eingesetzt werden.

Die Sichtbarkeit von Gewebestrukturen in der kernspinbasierten Bildgebung hängt unter anderem mit der Konzentration von Tracermolekülen am zu beobachtenden Ort, mit dem Grad der Polarisierung der Tracermoleküle und mit der T1-Relaxationszeit des Tracers zusammen. Eine höhere Konzentration von Tracermolekülen und/oder ein höherer Polarisierungsgrad der Tracermoleküle und/oder eine längere Tl-Relaxationszeit führt zu einer höheren Signalintensität im Magnetresonanzbild.

Die Aufgabe der Erfindung besteht darin, bei einem Einsatz eines Kontrastmittels der eingangs beschriebenen Art die Sichtbarkeit von Gewebestrukturen zu verbessern.

Zur Lösung der genannten Aufgabe sind erfindungsgemäß die Merkmale des Anspruchs 1 vorgesehen. Insbesondere wird somit zur Lösung der genannten Aufgabe bei Kontrastmitteln der eingangs beschriebenen Art erfindungsgemäß vorgeschlagen, dass das Kontrastmittel ein retardierendes Agens, welches in dieser Anmeldung als Retarder bezeichnet wird und aus Retarder-Molekülen besteht, aufweist, wobei eine molekulare Wechselwirkung von Retarder-Molekülen mit den Tracer-Molekülen eine Verlängerung der Tl-Relaxationszeit des Tracers zur Folge hat.

Die Erfindung hat erkannt, dass eine molekulare Wechselwirkung zwischen Retarder-Molekülen und Tracer-Molekülen genutzt werden kann, um die Tl-Relaxationszeit des Tracers zu verlängern.

Der Tracer und/oder der Retarder können aus neutralen, aber auch aus ionischen Molekülen bestehen. Sie können auch aus mehreren Molekülen zusammengesetzt sein und ein Addukt bilden. In diesem Fall ist es ausreichend, wenn ein molekularer Bestandteil als Tracer und/oder als Retarder wirkt.

Bei einer vorteilhaften Ausgestaltung kann vorgesehen sein, dass der polarisierbare Tracer hyperpolarisierbar ist und dass die Tracer-Moleküle durch Hyperpolarisation ihrer Kernspins in einen hyperpolarisierten Zustand bringbar sind.

Somit lassen sich Hyperpolarisationsverfahren, beispielsweise DNP, PHIP und/oder SABRE, auf das Kontrastmittel anwenden, um die Signalintensität des Tracers bei der kernspinbasierten Bildgebung zu erhöhen. Die Kernspins des Tracers können somit über ein thermisches Gleichgewicht hinaus geordnet ausgerichtet werden, wodurch ein größerer Grad der Polarisierung des Tracers erreicht werden kann, als im thermischen Gleichgewicht.

Bei einer vorteilhaften Ausgestaltung kann vorgesehen sein, dass der Tracer in einem Lösungsmittel gelöst und/oder dispergiert ist. Alternativ oder zusätzlich kann vorgesehen sein, dass der Retarder in einem Lösungsmittel gelöst und/oder dispergiert ist.

Somit sind der Tracer und/oder der Retarder in einer Flüssigkeit, beispielsweise eine flüssigen Kontrastmittel, handhabbar. Der Tracer und/oder der Retarder können hierbei molekular gelöst sein. Der Tracer und/oder der Retarder können mit dem Lösungsmittel auch eine Dispersion bilden, beispielsweise eine Suspension und/oder eine Emulsion. In diesem Fall bildet das Lösungsmittel das Dispersionsmedium zumindest anteilig. Je nach Eigenschaften des Tracers und/oder des Retarders können diese somit als Lösung und/oder als Dispersion in einen Patienten injizierbar sein.

Zusätzlich kann jeweils vorgesehen sein, dass das Lösungsmittel ausgewählt ist aus der Gruppe bestehend aus Wasser, Ethanol, Methanol, DMSO und deren Gemische.

Somit kann das Lösungsmittel je nach verwendetem Tracer und/oder Retarder vorteilhaft gewählt sein. Das Kontrastmittel kann neben dem Tracer, dem Retarder und dem Lösungsmittel, welches ein Dispersionsmedium darstellen und/oder zumindest anteilig bilden kann, auch weitere Zusätze, beispielsweise zur pH-Wert-Einstellung oder Stabilisierung einer Dispersion, enthalten.

Bei einer vorteilhaften Ausgestaltung kann vorgesehen sein, dass das Kontrastmittel eine die TI-Relaxationszeit des Tracers verkürzende Relaxationsquelle enthält, deren Wirkung durch die molekulare Wechselwirkung der Retarder-Moleküle mit den Tracer-Molekülen abgeschwächt und/oder unterdrückt wird.

Somit kann die molekulare Wechselwirkung des Retarders mit dem Tracers genutzt werden, um die Wirkung der Relaxationsquelle auf den Tracer und dessen TI-Relaxationszeit zu verringern. Bei der Relaxationsquelle kann es sich um eine Substanz handeln, die ohnehin, insbesondere notwendigerweise, in dem Kontrastmittel enthalten ist.

Zusätzlich kann vorgesehen sein, dass es sich bei der Relaxationsquelle um das oder ein Lösungsmittel handelt, vorzugsweise ausgewählt aus der Gruppe bestehend aus Wasser, Ethanol, Methanol, DMSO und deren Gemische.

Hierbei kann es sich um das Lösungsmittel handeln, in dem der Tracer und/oder Retarder gelöst und/oder dispergiert sind. Das Lösungsmittel kann somit auch als Dispersionsmedium fungieren und/oder dieses zumindest anteilig bilden. Somit kann der Retarder so gewählt werden, dass dieser die Wirkung eines die TI-Relaxationszeit des Tracers verkürzenden Lösungsmittels zumindest zum Teil kompensiert.

Alternativ und/oder zusätzlich kann vorgesehen sein, dass es sich bei der Relaxationsquelle um gelöste und/oder feste Substanzen, vorzugsweise Sauerstoff und/oder Verunreinigungen handelt.

Der Retarder kann somit so gewählt werden, dass dieser die Wirkung von die TI-Relaxationszeit des Tracers verkürzenden, gelösten und/oder festen Substanzen zumindest zum Teil kompensiert.

Bei einer vorteilhaften Ausgestaltung kann vorgesehen sein, dass die Wechselwirkung des Retarders mit dem Tracer stärker ist als eine Wechselwirkung der Relaxationsquelle mit dem Tracer.

Der Retarder kann daher so gewählt sein, dass dessen Wechselwirkung mit dem Tracer stärker ist als die der Relaxationsquelle. So kann der Retarder beispielsweise die Relaxationsquelle verdrängen und deren Einwirken auf den Tracer vermindern.

Zusätzlich kann vorgesehen sein, dass die Wechselwirkung des Retarders mit dem Tracer stärker ist als eine Wechselwirkung von Lösungsmittelmolekülen, insbesondere Wassermolekülen, mit dem Tracer. Alternativ oder zusätzlich kann vorgesehen sein, dass der Retarder eine Wechselwirkung zwischen der Relaxationsquelle und dem Tracer reduziert.

Je nach gewähltem Tracers und/oder Retarder kann die Art der Relaxationsquelle unerheblich in Bezug auf die Wechselwirkung des Retarders mit dem Tracer sein. Der Retarder kann beispielsweise dazu geeignet sein, die die TI-Relaxationszeit des Tracers verkürzende Wechselwirkung mehrerer unterschiedlicher Relaxationsquellen, beispielsweise sowohl ein Lösungsmittelgemisch als auch darin gelösten Sauerstoff und Verunreinigungen, mit dem Tracer zu reduzieren.

Bei einer vorteilhaften Ausgestaltung kann vorgesehen sein, dass die Wechselwirkung des Retarders mit dem Tracer zwischen den jeweiligen Retarder- und Tracer-Molekülen erfolgt.

Somit kann eine direkte Wechselwirkung zwischen Retarder und Tracer gegeben sein. Es ist jedoch auch möglich, dass der Retarder direkt mit einer Relaxationsquelle wechselwirkt, die folglich nicht mehr oder in einem geringeren Umfang als zuvor mit dem Tracer wechselwirken und seine Relaxation verursachen kann.

Alternativ oder zusätzlich kann vorgesehen sein, dass die Wechselwirkung des Retarders mit dem Tracer eine attraktive Wechselwirkung ist.

So kann beispielsweise eine Angriffsstelle einer Relaxationsquelle an den Tracer durch den Retarder blockiert und/oder abgeschirmt werden.

Alternativ oder zusätzlich kann vorgesehen sein, dass der Retarder eine Wechselwirkung zwischen dem Tracer und dem oder einem Lösungsmittel beeinflusst. Die Beeinflussung kann insbesondere eine Abschwächung sein.

Somit muss keine ausschließlich direkte Wechselwirkung zwischen dem Retarder und dem Tracer vorliegen, um beispielsweise eine Wechselwirkung zwischen einer Relaxationsquelle und dem Tracer zu vermindern oder zu unterbinden.

Bei einer vorteilhaften Ausgestaltung kann vorgesehen sein, dass es sich bei der molekularen Wechselwirkung von Retarder-Molekülen mit den Tracer-Molekülen und/oder der Relaxationsquelle mit dem Tracer und/oder der Lösungsmittelmoleküle mit dem Tracer um eine Wechselwirkung im Rahmen einer Wasserstoffbrückenbindung, eines hydrotropen Effekts, eines Molekülstoßes, einer Molekülbewegung, einer Molekülrotation, um elektromagnetische Wechselwirkungen und/oder um dipolare Wechselwirkungen handelt.

Die Wechselwirkung kann beispielsweise von einem einzelnen Atom eines Moleküls ausgehen. Hierbei kann die Wechselwirkung beispielsweise hauptsächlich auf ein freies Elektronenpaar zurückzuführen sein. Die Wechselwirkung kann auch von delokalisierten Elektronen ausgehen. Dieselbe Wechselwirkung kann somit auch mehreren Atomen desselben Moleküls zugeschrieben werden. Unter dem Ausgehen einer Wechselwirkung kann im Sinne dieser Anmeldung insbesondere verstanden werden, dass die Struktur, von der die Wechselwirkung ausgeht, an der Wechselwirkung beteiligt ist. Hierbei kann an der Struktur, also beispielsweise einem Atom und/oder delokalisierten Elektronen und/oder den delokalisierten Elektronen zugeordneten Atomen, eine resultierende Kraft der Wechselwirkung anliegen.

Zusätzlich kann vorgesehen sein, dass die molekulare Wechselwirkung der Relaxationsquelle mit dem Tracer und/oder der Lösungsmittelmoleküle mit dem Tracer die Wechselwirkung die Tl-Relaxationszeit des Tracers verkürzt.

Es ist in diesem Zusammenhang von besonderem Vorteil, wenn diese die Tl-Relaxationszeit des Tracers verkürzende Wechselwirkung durch den Retarder vermindert oder unterbunden wird.

Bei einer vorteilhaften Ausgestaltung kann vorgesehen sein, dass der Retarder eine Bindungsstelle, insbesondere mehrere Bindungsstellen, des Tracers räumlich abschirmt. Hierbei handelt es sich um eine Bindungsstelle, über die die oder eine Relaxationsquelle mit dem Tracer wechselwirken kann und/oder um eine Bindungsstelle, über die die oder eine Relaxationsquelle eine Verkürzung der Tl-Relaxationszeit des Tracers bewirken kann. Bei der Relaxationsquelle kann es sich beispielsweise um das oder ein Lösungsmittelmolekül handeln.

Somit kann der Tracer von einer Relaxationsquelle durch den Retarder beispielsweise durch sterische Hinderung abgeschirmt werden, um die von der Relaxationsquelle verursachte Verkürzung der Tl-Relaxationszeit zu vermindern oder zu unterbinden.

Bei einer vorteilhaften Ausgestaltung kann vorgesehen sein, dass eine durchschnittliche Dauer einer Wechselwirkung zwischen dem Retarder und dem Tracer 10 ms übersteigt.

Somit kann die Wechselwirkung zwischen dem Retarder und dem Tracer Relaxationsquellen lange genug von einer die T1-Relaxationszeit des Tracers verkürzenden Wechselwirkung mit diesem abhalten, um eine ausreichende Signalstärke und Bildqualität in der kernspinbasierten Bildgebung zu erreichen.

Alternativ oder zusätzlich kann vorgesehen sein, dass das Kontrastmittel in einem Magnetfeld eingebracht ist und dass eine durchschnittliche Dauer einer Wechselwirkung zwischen dem Retarder und dem Tracer ein Vielfaches einer Periode einer von dem Magnetfeld ausgelösten Larmorfrequenz eines hyperpolarisierten Atomkerns des Tracers übersteigt. Das Vielfache ist wenigstens das 100-fache, bevorzugt das 1000-fache, das 10.000-fache, das 100.000-fache, das 300.000-fache oder das 1.000.000-fache.

Bei einer vorteilhaften Ausgestaltung kann vorgesehen sein, dass mehrere Wechselwirkungen von Atomen eines Retarder-Moleküls ausgehen.

Bei den mehreren Wechselwirkungen handelt es sich vorzugsweise um gleichartige Wechselwirkungen. So kann es sich beispielsweise bei den mehreren Wechselwirkungen jeweils um ionische Wechselwirkungen und/oder Wasserstoffbrücken handeln. Vorzugsweise gehen die mehreren Wechselwirkungen von verschiedenen Atomen des Retarder-Moleküls aus. Die mehreren Wechselwirkungen können jedoch auch beispielsweise von delokalisierten Elektronen ausgehen.

Alternativ oder zusätzlich kann vorgesehen sein, dass mehrere Wechselwirkungen an Atomen eines oder mehrerer Tracer-Moleküle angreifen.

Hierbei handelt es sich vorzugsweise um gleichartige Wechselwirkungen. Die mehreren Wechselwirkungen können vorzugsweise an verschiedenen Atomen des oder der TracerMoleküle angreifen. Unter einem Angreifen einer Wechselwirkung im Sinne dieser Anmeldung kann insbesondere verstanden werden, dass die Struktur, beispielsweise das Atom, an der Wechselwirkung beteiligt ist. Hierbei kann an der Struktur eine resultierende Kraft der Wechselwirkung anliegen.

Beispielsweise können zwischen einem Tracer-Molekül und einem Retarder-Molekül zwei Wechselwirkungen in Form von Wasserstoffbrückenbindungen bestehen, wobei jeweils eines der beteiligten Wasserstoffatome von dem Tracer-Molekül und eines von dem Retarder-Molekül bereitgestellt wird und an dem jeweils anderen Molekül mit einem Stickstoffatom wechselwirken. In diesem Fall gehen somit im Sinne dieser Anmeldung mehrere gleichartige Wechselwirkungen von verschiedenen Atomen des Retarder-Moleküls aus und greifen an verschiedenen Atomen des Tracer-Moleküls an.

Bei einer vorteilhaften Ausgestaltung kann vorgesehen sein, dass der Tracer ein vorzugsweise wasserlösliches Biomolekül ist.

Somit kann die Erfindung auch mit Biomolekülen als Tracer verwendet werden. Die Wasserlöslichkeit des Tracers verbessert seine Handhabbarkeit in biokompatiblen Lösungen, seine Verteilung im Körper des Patienten und seinen Abbau bzw. seine Ausscheidung aus dem Körper. Diese Vorteile lassen sich bereits realisieren, wenn der Tracer lediglich wasserlöslich, jedoch kein Biomolekül ist. Auch dies ist Teil dieser Erfindung.

Alternativ oder zusätzlich kann vorgesehen sein, dass der Tracer eine Struktur ausgewählt aus der Gruppe bestehend aus $^{13}$C-Atom, $^{15}$N-Atom, $^{29}$Si-Atom, $^{31}$P-Atom, $^{13}$C-Carbonylgruppe, $^{15}$N-Pyridinring aufweist.

Somit kann der Tracer unterschiedliche Atomkerne zur Polarisierung bzw. Hyperpolarisierung bereitstellen, um eine vielfältige kernspinbasierte Bildgebung zu ermöglichen.

Bei einer vorteilhaften Ausgestaltung kann vorgesehen sein, dass es sich bei dem Tracer und/oder dem Retarder um einen Stoff ausgewählt aus der Gruppe bestehend aus Nicotinamid, Nicotinsäure und Succinate handelt.

Somit können eine Reihe von Biomolekülen mit der Erfindung bzw. in der kernspinbasierten Bildgebung verwendet werden. Die Biomoleküle können insbesondere am Stoffwechsel des Patienten teilnehmen, um Gebiete erhöhter Stoffwechselaktivität anzuzeigen. Die Biomoleküle können zur Herstellung einer Biokompatibilität des Kontrastmittels dienen.

Zusätzlich kann vorgesehen sein, dass der Tracer stofflich dem Retarder entspricht.

Der Tracer und der Retarder können sich dennoch atomar unterscheiden, nämlich unterschiedliche Isotope desselben Elements aufweisen. Es ist daher nicht erforderlich, dass nicht nur der Tracer, sondern auch der Retarder einen Kernspin aufweist. So kann beispielsweise nur der Tracer, jedoch nicht der Retarder in der kernspinbasierten Bildgebung darstellbar sein.

Bei einer vorteilhaften Ausgestaltung kann vorgesehen sein, dass es sich bei dem Retarder um einen Stoff ausgewählt aus der Gruppe bestehend aus Harnstoff, Thioharnstoff, Sulfaten, Cystein, Cystin, Amiden, Glycerinen, Dendrimeren von vorzugsweise einer zweiten Generation, Triphenylboran, Lewis-Säuren, insbesondere mit Bor als Lewis-saurem Zentrum, Metallen und Wasserstoffbrückendonoren handelt. Insbesondere bei Verwendung von Bor ist es von Vorteil, das Quadrupolmoment der natürlich vorkommenden Isotope $^{10}B$ und $^{11}B$ zu beachten.

Somit kann der Retarder aus einer Reihe von Molekülen, beispielsweise Biomolekülen und/oder Stoffwechselmetaboliten, ausgewählt werden. Der Retarder kann beispielsweise so gewählt werden, dass dieser eine Bindung einer gewünschten Stärke mit dem Tracer eingeht, und zu einer gewünschten Verlängerung der Tl-Relaxationszeit führt.

Bei einer vorteilhaften Ausgestaltung kann vorgesehen sein, dass es sich bei dem Lösungsmittel um Wasser handelt und Tracer und Retarder ausgewählt sind aus folgenden Tracer/Retarder-Paaren: Nicotinamid/Harnstoff, Nicotinamid/Nicotinamid, Pyridin/Nicotinamid, Pyridin/Harnstoff, Pyrimidin/Nicotinamid, Pyrimidin/Harnstoff oder Metronidazol/Harnstoff, Thioharnstoff/Pyridin, Glucose/Pyridin, Ascorbinsäure/Pyridin, Milchsäure/Pyridin, Pyridin/Triphenylboran.

Das Kontrastmittel kann somit Wasser als Lösungsmittel aufweisen und insbesondere ohne Entmischung in einen Patienten injiziert werden. Das Kontrastmittel kann weitere Lösungsmittel aufweisen. Die ausgewählten Tracer/Retarder-Paare weisen eine besonders vorteilhafte Wechselwirkung zwischen Tracer und Retarder und/oder zwischen Retarder und Relaxationsquellen auf, so dass die Tl-Relaxationszeit des Tracers vorteilhaft verlängert wird. Die Verwendung von Ascorbinsäure kann einen Sauerstoffanteil in dem Lösungsmittel reduzieren, so dass dieser nur in einem reduzierten Maße als Relaxationsquelle wirken kann.

Bei einer vorteilhaften Ausgestaltung kann vorgesehen sein, dass zumindest der Tracer hyperpolarisiert ist.

Somit ist es möglich, ein hyperpolarisiertes Kontrastmittel bereitzustellen und eine noch höhere Signalintensität und damit bessere Qualität in der kernspinbasierten Bildgebung zu erreichen.

Zusätzlich kann vorgesehen sein, dass zumindest der Tracer mittels DNP, PHIP und/oder SABRE hyperpolarisiert ist.

Der Tracer des Kontrastmittels kann mittels unterschiedlicher, bereits etablierter Methoden hyperpolarisiert sein.

Alternativ oder zusätzlich kann vorgesehen sein, dass eine Konzentration des Retarders größer als eine Konzentration des Tracers ist.

So kann die Konzentration des Retarders an die des Tracers angepasst sein. Bei der Konzentration kann es sich beispielsweise um eine Massenkonzentration und/oder eine Stoffmengenkonzentration handeln. Durch eine höhere, insbesondere mehrfach höhere, Stoffmengenkonzentration des Retarders als des Tracers kann beispielsweise sichergestellt werden, dass wenigstens ein Retardermolekül pro Tracermolekül zur Verfügung steht.

Alternativ oder zusätzlich kann vorgesehen sein, dass eine Konzentration der Bindungsstellen des Retarders größer als eine Konzentration der Bindungsstellen des Tracers ist.

Bei den Bindungsstellen kann es sich beispielsweise um Wasserstoffbrückendonor- und/oder -akzeptoratome handeln. Die Konzentration der Bindungsstellen kann beispielsweise analog einer Stoffmengenkonzentration in mol/L ausgedrückt werden.

Durch eine höhere, insbesondere mehrfach höhere, Konzentration der Bindungsstellen des Retarders kann beispielsweise sichergestellt werden, dass die Bindungsstellen des Tracers mit denen des Retarders gesättigt sind. Es könnte in diesem Zusammenhang von einer Art kompetitiver Hemmung der Bindungsstellen des Tracers durch die des Retarder gesprochen werden. An den Bindungsstellen des Tracers angreifende Relaxationsquellen werden somit an ihrem Einfluss auf den Tracer gehindert und die T1-Relaxationszeit des Tracers kann verlängert sein.

Alternativ oder zusätzlich kann vorgesehen sein, dass eine Konzentration des Retarders größer als 100 mM ist.

Somit ist die Konzentration des Retarders derart festlegbar, dass diese 100 mM übersteigt. Wird die Konzentration des Tracers geringer, insbesondere mehrfach geringer, als die des Retarders gewählt, kann der Tracer kompetitiv gegenüber Relaxationsquellen abgeschirmt sein.

Die Konzentration des Retarders ist auch dadurch festlegbar, dass ein Gleichgewicht zwischen der Abschirmung des Tracers gegenüber Relaxationsquellen durch den Retarder, wobei die T1-Relaxationszeit des Tracers verlängert werden kann, und einer die Tl-Relaxationszeit des Tracers verkürzenden, zu hohen Konzentration des Retarders, ermittelt und eingestellt wird.

Alternativ oder zusätzlich sind zur Lösung der genannten Aufgabe erfindungsgemäß die Merkmale des nebengeordneten, auf ein Verfahren zur Herstellung eines hyperpolarisierten Kontrastmittels gerichteten Anspruchs vorgesehen. Insbesondere wird somit zur Lösung der genannten Aufgabe bei Verfahren der eingangs beschriebenen Art erfindungsgemäß vorgeschlagen, dass es sich bei dem hyperpolarisierten Kontrastmittel um ein Kontrastmittel nach einem der vorangehenden Ansprüche zumindest mit hyperpolarisiertem Tracer handelt, wobei die Hyperpolarisation mittels DNP, PHIP und/oder SABRE erfolgt und wobei der Retarder vor, während und/oder nach der Hyperpolarisation zugegeben wird.

Somit kann ein erfindungsgemäßes Kontrastmittel als hyperpolarisiertes Kontrastmittel bereitgestellt werden. Dieses weist gegenüber einem nicht hyperpolarisierten Kontrastmittel einen größeren, ein thermisches Gleichgewicht übersteigenden, Anteil von ausgerichteten Kernspins und somit eine größere Polarisierung auf. Das hyperpolarisierte Kontrastmittel führt zu einem stärkeren Signal und damit einer besseren Bildqualität in der kernspinbasierten Bildgebung.

Alternativ oder zusätzlich sind zur Lösung der genannten Aufgabe erfindungsgemäß die Merkmale des nebengeordneten, auf einen Retarder zur Verwendung in einem hyperpolarisierten Kontrastmittel zur bildgebenden Darstellung eines Stoffwechselprozesses gerichteten Anspruchs vorgesehen. Bei dem Kontrastmittel kann es sich insbesondere um ein erfindungsgemäßes Kontrastmittel handeln. Bei dem Kontrastmittel kann es sich insbesondere auch um ein in einem erfindungsgemäßen Verfahren hergestelltes Kontrastmittel handeln. Insbesondere wird somit zur Lösung der genannten Aufgabe bei Retardern der eingangs beschriebenen Art erfindungsgemäß vorgeschlagen, dass der Retarder mit dem hyperpolarisierten Tracer des Kontrastmittels wechselwirkt, wodurch eine Tl-Relaxationszeit des hyperpolarisierten Tracers verlängert wird.

Somit kann ein Retarder hergestellt werden, der in einem Kontrastmittel verwendbar ist. Beispielsweise kann das Kontrastmittel industriell bereitgestellt werden und der Retarder anschließend hinzugegeben werden, um die T1-Relaxationszeit eines Inhaltsstoffes des Kontrastmittels zu verlängern. Beispielsweise kann ein Retarder für unterschiedliche Arten von Kontrastmitteln verwendbar sein. Bei einer vorteilhaften Ausgestaltung kann vorgesehen sein, dass der Retarder zur Anwendung in einem diagnostischen Verfahren in vivo und/oder in vitro vorgesehen ist. Der Retarder kann insbesondere zur Detektion von Erkrankungen, Überwachung von Krankheitsverläufen und/oder des Therapieansprechens, insbesondere bei Tumor-Erkrankungen und/oder metabolischen Erkrankungen, vorgesehen sein.

Somit können die Vorteile der Erfindung in einem derartigen Verfahren genutzt werden.

Alternativ oder zusätzlich sind zur Lösung der genannten Aufgabe erfindungsgemäß die Merkmale des nebengeordneten, auf ein Kontrastmittel zur Anwendung in einem diagnostischen Verfahren in vivo und/oder in vitro gerichteten Anspruchs vorgesehen. Insbesondere wird somit zur Lösung der genannten Aufgabe bei Kontrastmitteln der eingangs beschriebenen Art erfindungsgemäß vorgeschlagen, dass es sich bei dem Kontrastmittel um ein erfindungsgemäßes Kontrastmittel wie zuvor beschrieben handelt und/oder dass das Kontrastmittel ein nach einem erfindungsgemäßen Verfahren hergestelltes Kontrastmittel ist.

Zusätzlich kann das Kontrastmittel zur Anwendung in einem diagnostischen Verfahren in vivo und/oder in vitro zur Detektion von Erkrankungen, Überwachung von Krankheitsverläufen und/oder des Therapieansprechens, insbesondere bei Tumor-Erkrankungen und/oder metabolischen Erkrankungen, vorgesehen sein.

Somit können die Vorteile erfindungsgemäßer und erfindungsgemäß hergestellter Kontrastmittel in derartigen Verfahren genutzt werden.

Eine bevorzugte Anwendung der Erfindung sieht vor, dass in einem hyperpolarisierbaren Kontrastmittel ein erfindungsgemäßer Retarder zur Verlängerung der T1-Relaxationszeit des Tracers verwendet wird.

Somit können die Vorteile erfindungsgemäßer Retarder bei der Anwendung von beispielsweise separat hergestellten Kontrastmitteln genutzt werden.

Die Erfindung wird nun anhand eines Ausführungsbeispiels näher beschrieben, ist jedoch nicht auf das Ausführungsbeispiel beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigen:

Figur 1     die Polarisation bzw. einen Polarisationsgrad sowie eine Tl-Relaxationszeit verschiedener Atome von 1-$^{15}$N-Nicotinamid in Abhängigkeit von einer Retarderkonzentration,

Figur 2     die Polarisation bzw. einen Polarisationsgrad sowie eine Tl-Relaxationszeit von $^{15}$N-Atomen verschiedener Tracer in verschiedenen Umgebungen mit und ohne Retarder.

Zur Darlegung des Effekts der Erfindung wurden Proben zur Hyperpolarisierung mittels dDNP durch Mischung eines Substrats mit Triphenylmethylradikal (Tritylradikal) in entionisiertem Wasser mit Trehalose vorbereitet. Als Substrat wurde 1-$^{15}$N-Nicotinamid (NAM), $^{15}$N-Pyridin, Metronidazol (MTZ), $^{15}N_2$-Harnstoff oder Pyrimidin verwendet. Hieraus ergeben sich Ausführungsbeispiele wie folgt:

130 mg Wasser und 60 mg Trehalose, 100 mg 1-$^{15}$N-NAM, und
10.5 mg Tritylradikal, wobei ein Probenvolumen von 225 μL mit etwa 29 mM Tritylradikal und 3.6 M 1-$^{15}$N-NAM erhalten wird.
100 mg Wasser und 48 mg Trehalose, 68 mg $^{15}$N-Pyridin, und
8.1 mg Tritylradikal, wobei ein Probenvolumen von 185 μL mit etwa 27 mM Tritylradikal und 4.6 M $^{15}$N-Pyridin erhalten

wird.

300 mg DMSO, 77 mg MTZ, und 11.4 mg Tritylradikal, wobei ein Probenvolumen von 335 µL mit etwa 21.3 mM Tritylradikal und 1.34 M MTZ erhalten wird.

49 mg Trehalose, 108 mg Pyrimidin, und 5.8 mg Tritylradikal, wobei ein Probenvolumen von 144 µL mit etwa 25 mM Tritylradikal und 9.3 M Pyrimidin erhalten wird.

Nach der Herstellung wurde die Lösung bei -24°C gelagert. Vor der Verwendung wurde das Fläschchen erwärmt (in den Händen) und 2 Minuten lang mit einem Vortexmischer gemischt. Die typische Probengröße betrug 30 mg für Pyridin und 50 mg für die anderen Substanzen. 1-$^{15}$N-NAM wurde in einer zweistufigen Reaktion aus NAM (72340, CAS: 98-92-0, Sigma-Aldrich) über das Zincke-Salz synthetisiert, gefolgt von einem Stickstoffaustausch mit $^{15}$NH$_4$Cl(299251, CAS: 39466-62-1, Sigma-Aldrich). Im ersten Schritt wurde das Zincke-Salz von NAM mit 1-Chlor-2,4-dinitrobenzol (237329, CAS: 97-00-7, Sigma-Aldrich) in DMSO gebildet. Die resultierende Verbindung war ein leicht gelbliches Pulver, das anschließend mit $^{15}$NH$_4$Cl umgesetzt wurde, um 1-$^{15}$N-NAM als weißes Pulver zu erhalten. In einigen Fällen zeigte sich nach der chromatographischen Aufarbeitung eine gelbe Färbung durch die Anwesenheit von 2,4-Dinitroanilin, das chromatographisch nicht abgetrennt werden konnte. In diesen Fällen kann beispielsweise ein zusätzlicher Reinigungsschritt mit Aktivkohle vor der säulenchromatographischen Aufarbeitung oder eine zusätzliche Umkristallisation in Ethylacetat nach der Säulenchromatographie erfolgen.

Das Auflösungsmedium (dissolution medium) mit einem pH-Wert von 7,5 wurde durch Mischen von 300 mg Trizma pre-set crystals (pH-Wert 7,6, durchschnittliches M = 149,0 g/mol, T7943, Sigma-Aldrich) und 50 mg Ethylendiamintetraessigsäure (EDTA, 11280, CAS: 9002-07-7, SERVA) in 50 mL entionisiertem Wasser oder 99,9 % D$_2$O(151882, Sigma-Aldrich) hergestellt und bei Raumtemperatur gelagert. Dem Medium kann beispielsweise NAM mit natürlicher Isotopenverteilung (n.a.) (72340, CAS: 98-92-0, Sigma-Aldrich), Bipyridin (CDS018251, Sigma-Aldrich) und Harnstoff (2317, CAS: 57-13-6, Carl Roth) zugesetzt werden. Um ein Auflösungsmedium mit einem pH-Wert von 8,5 zu erhalten, wurden etwa 50 mg NaOH (1355, CAS: 1310-73-2, ChemSolute) hinzugefügt.

Alle dDNP-Experimente wurden mit einem kryogenfreien dDNP-System (SpinAligner) bei ~1,4 K und 6,7 T durchgeführt. Zur Polarisierung wurde eine Mikrowellenfrequenz (MW) zwischen 187,07 und 187,19 GHz mit 10 bis 45 mW Leistung verwendet. Die optimale MW-Frequenz wurde für jede Probencharge durch Variation der Mikrowellenfrequenz kalibriert. Für jedes DNP-Experiment wurde die angegebene Menge des Konzentrats (in der Regel 50 mg) aus dem Vorrat entnommen, in den Probenbecher gefüllt und bei $\approx$ 1,3 K in die Mikrowellenkavität gesenkt. Die DNP wurde durch kontinuierliche Wellenbestrahlung bei der optimierten Frequenz und Leistung eingeleitet. Der Aufbau der $^{15}$N-Polarisation im festen Zustand wurde alle 15 Minuten mit einem $^{15}$N-HF-Puls von 3° überwacht. Der Flipping-Winkel des eingebauten NMR wurde vor den Experimenten auf $^{15}$N kalibriert.

$^{15}$N-MR-Signale wurden mit zwei 1-T-$^{13}$C- und $^{15}$N-Benchtop-NMR-Geräten (Spinsolve Nitrogen, Magritek), einem 9,4-T-wide-bore-NMR-Gerät (WB400, Avance NEO, Bruker) mit einer 5-mm-BBFO-Sonde und einem 0,57-T-10-mm-Tisch-MRT-System (Magneteinheit "magspec", Konsoleneinheit "drive L", Pure Devices GmbH) erfasst.

Für den Transport zu den Messplätzen bei 0,57 T und 9,4 T wurde ein 0,5-1 T Halbach-Magnet in an sich bekannter Weise verwendet.

Die NMR-Spektren wurden durch manuelle Integration nach manueller Phasenkorrektur, Linienverbreiterung und Basislinienkorrektur quantifiziert (MestReNova 14.2.2, Mestrelab Research S.L.). Die MRT-Bilder wurden mit der Software des Herstellers (Paravision 360, Bruker) ausgewertet.

Die Signalverstärkung wurde mit Hilfe von (Gl. 1) in Bezug auf das akkumulierte Signal der gleichen Probe im thermischen Gleichgewicht quantifiziert:

$$P^{HP} = P^{TP} \cdot \varepsilon = \frac{S^{\mathrm{HP}}}{S^{\mathrm{TP}}} \cdot \frac{NS_{\mathrm{acq}}^{\mathrm{TP}}}{NS_{\mathrm{acq}}^{\mathrm{HP}}} \cdot \frac{\sin(\alpha^{\mathrm{TP}})}{\sin(\alpha^{\mathrm{HP}})} \cdot \frac{RG^{\mathrm{TP}}}{RG^{\mathrm{HP}}}, \qquad (\mathrm{Gl.\ 1})$$

wobei $P^{TP}$ die Polarisation im thermischen Gleichgewicht, S$^x$ das Integral des jeweiligen Signals, $NS_{\mathrm{acq}}^{\mathrm{x}}$ die Anzahl der akkumulierten Spektren, $\alpha^x$ der Anregungswinkel und $RG^x$ die lineare Empfängerverstärkung für hyperpolarisierte (x = HP) und thermisch polarisierte (x = TP) NMR-Spektren sind.

Thermisch polarisierte Flüssigzustand-NMR-Spektren wurden mit hochauflösender NMR bei 9,4 T, $^1$H-Ent-kopplung und den folgenden Aufnahmeparametern aufgenommen: Anzahl der Akkumulationen $NS_{\mathrm{acq}}^{\mathrm{TP}}$ = 64, Flip-Winkel $\alpha^{TP}$ = 90°, Wiederholungszeit TR = 170 s. Es wurde ein typisches Signal-Rausch-Verhältnis von 20 erzielt. Die $^{15}$N-Signalintensitäten wurden mit Hilfe der Quantifizierungsmethoden quantifiziert, wobei der ausgewählte Integrationsbereich um das hyperpolarisierte Signal $\pm 3$ ppm betrug. Die Polarisation wurde nur bei 9,4 T quantifiziert.

Die abklingende Hyperpolarisation wurde mit einem $\alpha^{HP}$ = 5°-10°-Puls alle 3-6 s abgetastet. Zur Quantifizierung der Lebensdauer der Hyperpolarisation $T_1^{\mathrm{HP}}$ wurde eine monoexponentielle Abklingfunktion an die Daten angepasst, die die scheinbare/beobachtete Konstante $T_1^{\mathrm{obs}}$ ergibt (Gl. 2):

$$S^{\mathrm{obs}}(t) = S_0 \cdot e^{-\frac{t}{T_1^{\mathrm{obs}}}}. \qquad \qquad (\mathrm{Eq.\ 2})$$

Um die longitudinale Relaxationszeit T1 zu erhalten, wurde $T_1^{\mathrm{obs}}$ in allen Fällen, wenn nicht anders angegeben, korrigiert, wobei die durch die wiederholten HF-Anregungen mit dem Winkel $\alpha^{\mathrm{HP}}$ konsumierte Polarisation berücksichtigt wurde:

$$T_1 = T_1^{\mathrm{obs}}\left[1 + \frac{T_1^{\mathrm{obs}}}{TR}\ln\left(\cos(\alpha^{\mathrm{HP}})\right)\right]^{-1} \qquad \qquad (\mathrm{Eq.\ 3})$$

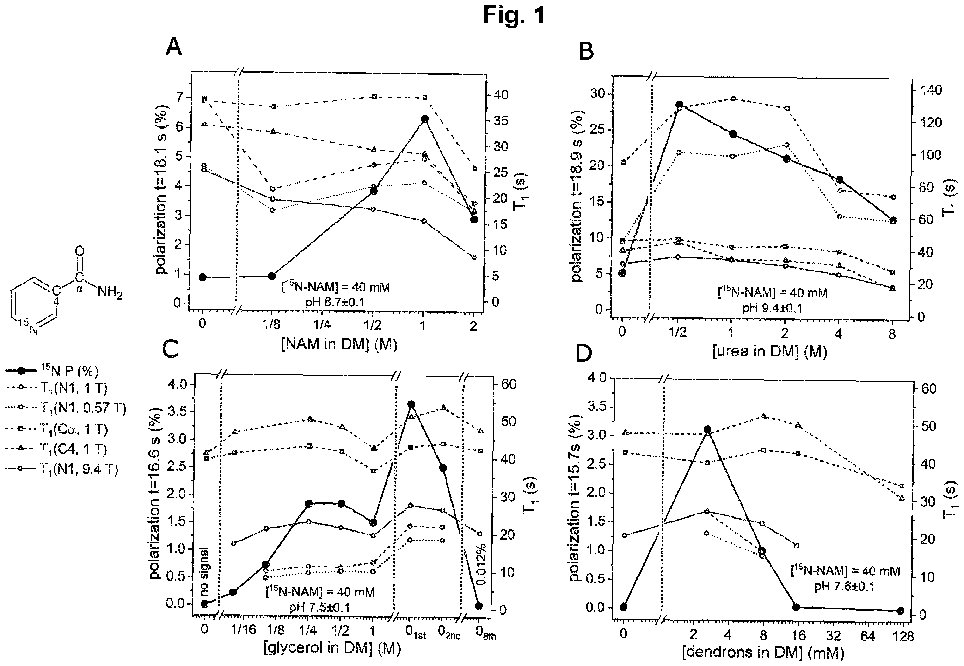

Figur 1 zeigt die Polarisation bzw. einen Polarisationsgrad (polarization in %) sowie eine TI-Relaxationszeit ($T_1$ in Sekunden) der in der Strukturformel dargestellten Atome $^{15}$N, $C_\alpha$, C4 und N1 (Stickstoff der Aminogruppe) in Abhängigkeit von der Konzentration der Retarder in natürlicher

Isotopenverteilung: NAM (A), Harnstoff (urea) (B), Glycerin (glycerol) (C) und eines Dendrimers (dendrons) (D). Vor allem relevant sind die dargestellten Daten für $^{15}$N, welches Teil des Tracers ist und in der Bildgebung nachgewiesen werden soll. Die übrigen Daten dienen als Kontrolle. Dargestellt sind ferner die Konzentration von $^{15}$N-NAM und der pH-Wert der jeweiligen Probe. Es ist erkennbar, dass die Zugabe der Retarder zu einer um ein Vielfaches besseren Signalerhaltung führt. Die Polarisation bzw. die TI-Relaxationszeit ist deutlich erhöht. Diese Erhöhung kann bei zugegebenem Retarder auch ohne erneute Zugabe des Retarders über mehrere Zyklen des Experiments erhalten bleiben (siehe (C), erste ($O_{1\mathrm{st}}$), zweite ($0_{2\mathrm{nd}}$) und achte ($0_{8\mathrm{th}}$) Wiederholung) .

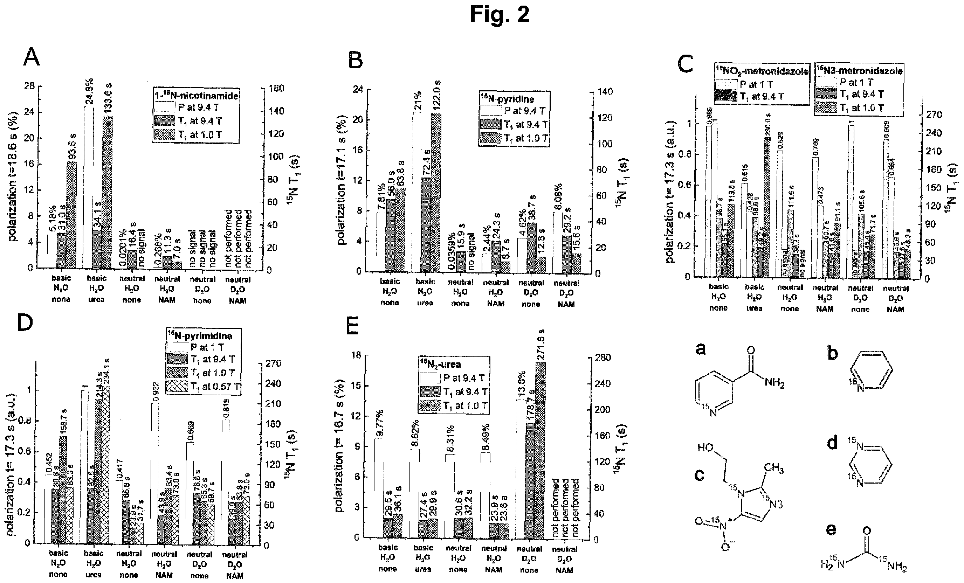

Figur 2 zeigt die Polarisation bzw. einen Polarisationsgrad (polarization in %) sowie eine TI-Relaxationszeit ($T_1$ in Sekunden) von in Strukturformeln a bis e dargestellten $^{15}$N-Atomen verschiedener Tracer in verschiedenen Umgebungen mit und ohne Retarder. Für 1-$^{15}$N-NAM (A), $^{15}$N-Pyridin (B), $^{15}$NO$_2$-Metronidazol und $^{15}$N3-Metronidazol (C), $^{15}$N-Pyrimidin (D) und $^{15}$N-Harnstoff ist für basische (basic) und neutrale Umgebung in Wasser bzw. schwerem Wasser und mit oder ohne Retarder in Form von Harnstoff oder NAM dargestellt, dass sowohl Polarisation als auch T1-Relaxationszeit deutlich erhöht werden können.

Es wird somit ein Kontrastmittel für die kernspinbasierte Bildgebung vorgeschlagen, mit einem einen Kernspin aufweisenden, polarisierbaren Agens (Tracer), wobei Tracer-Moleküle durch Polarisation ihrer Kernspins in einen polarisierten Zustand bringbar sind, und mit einem retardierenden Agens (Retarder), wobei eine molekulare Wechselwirkung von Retarder-Molekülen mit den Tracer-Molekülen eine Verlängerung der T1-Relaxationszeit des Tracers zur Folge hat. Ferner werden ein Verfahren zur Herstellung eines hyperpolarisierten Kontrastmittels, ein Retarder zur Verwendung in einem Kontrastmittel für die kernspinbasierte Bildgebung, ein Kontrastmittel zur Anwendung in einem diagnostischen Verfahren in vivo und/oder in vitro, und die Verwendung eines Retarders in einem Kontrastmittel für die kernspinbasierte Bildgebung zur Verlängerung der T1-Relaxationszeit des Tracers vorgeschlagen.

## Patentansprüche

1. Kontrastmittel für die kernspinbasierte Bildgebung, mit einem einen Kernspin aufweisenden, polarisierbaren Agens (Tracer), wobei Tracer-Moleküle durch Polarisation ihrer Kernspins in einen polarisierten Zustand bringbar sind, und mit einem retardierenden Agens (Retarder), wobei eine molekulare Wechselwirkung von Retarder-Molekülen mit den Tracer-Molekülen eine Verlängerung der TI-Relaxationszeit des Tracers zur Folge hat.

2. Kontrastmittel nach dem vorangehenden Anspruch, **dadurch gekennzeichnet, dass** der polarisierbare Tracer hyperpolarisierbar ist und dass die Tracer-Moleküle durch Hyperpolarisation ihrer Kernspins in einen hyperpolarisierten Zustand bringbar sind.

3. Kontrastmittel nach einem der vorangehenden Ansprüche, **dadurch gekennzeichnet, dass** der Tracer und/oder der Retarder in einem Lösungsmittel gelöst und/oder dispergiert sind/ist, insbesondere wobei das Lösungsmittel ausgewählt ist aus der Gruppe bestehend aus Wasser, Ethanol, Methanol, DMSO und deren Gemische.

4. Kontrastmittel nach einem der vorangehenden Ansprüche, **dadurch gekennzeichnet, dass** das Kontrastmittel eine die T1-Relaxationszeit des Tracers verkürzende Relaxationsquelle enthält, deren Wirkung durch die molekulare Wechselwirkung der Retarder-Moleküle mit den Tracer-Molekülen abgeschwächt und/oder unterdrückt wird, insbesondere wobei es sich bei der Relaxationsquelle um das oder ein Lösungsmittel handelt, vorzugsweise ausgewählt aus der Gruppe bestehend aus Wasser, Ethanol, Methanol, DMSO und deren Gemische, und/oder wobei es sich bei der Relaxationsquelle um gelöste und/oder feste Substanzen, vorzugsweise Sauerstoff und/oder Verunreinigungen handelt.

5. Kontrastmittel nach einem der vorangehenden Ansprüche, **dadurch gekennzeichnet, dass** die Wechselwirkung des Retarders mit dem Tracer stärker ist als eine Wechselwirkung der Relaxationsquelle mit dem Tracer, insbesondere wobei die Wechselwirkung des Retarders mit dem Tracer stärker ist als eine Wechselwirkung von Lösungsmittelmolekülen, insbesondere Wassermolekülen, mit dem Tracer, und/oder dass der Retarder eine Wechselwirkung zwischen der Relaxationsquelle und dem Tracer reduziert.

6. Kontrastmittel nach einem der vorangehenden Ansprüche, **dadurch gekennzeichnet, dass** die Wechselwirkung des Retarders mit dem Tracer zwischen den jeweiligen Retarder- und Tracer-Molekülen erfolgt und/oder eine attraktive Wechselwirkung ist, und/oder dass der Retarder eine Wechselwirkung zwischen dem Tracer und dem oder einem Lösungsmittel beeinflusst, insbesondere abschwächt.

7. Kontrastmittel nach einem der vorangehenden Ansprüche, **dadurch gekennzeichnet, dass** es sich bei der molekularen Wechselwirkung von Retarder-Molekülen mit den Tracer-Molekülen und/oder der Relaxationsquelle mit dem Tracer und/oder der Lösungsmittelmoleküle mit dem Tracer um eine Wechselwirkung im Rahmen einer Wasserstoffbrückenbindung, eines hydrotropen Effekts, eines Molekülstoßes, einer Molekülbewegung, einer Molekülrotation, um elektromagnetische Wechselwirkungen und/oder um dipolare Wechselwirkungen handelt, vorzugsweise wobei die Wechselwirkung der Relaxationsquelle mit dem Tracer und/oder der Lösungsmittelmoleküle mit dem Tracer die T1-Relaxationszeit des Tracers verkürzt.

8. Kontrastmittel nach einem der vorangehenden Ansprüche, **dadurch gekennzeichnet, dass** der Retarder eine Bindungsstelle, insbesondere mehrere Bindungsstellen, des Tracers, über die die oder eine Relaxationsquelle mit dem Tracer wechselwirken und/oder eine Verkürzung seiner T1-Relaxationszeit bewirken kann, räumlich abschirmt.

9. Kontrastmittel nach einem der vorangehenden Ansprüche, **dadurch gekennzeichnet, dass** eine durchschnittliche Dauer einer Wechselwirkung zwischen dem Retarder und dem Tracer 10 ms übersteigt und/oder dass das Kontrastmittel in einem Magnetfeld eingebracht ist und dass eine durchschnittliche Dauer einer Wechselwirkung zwischen dem Retarder und dem Tracer eine Periode einer von dem Magnetfeld ausgelösten Larmorfrequenz eines hyperpolarisierten Atomkerns des Tracers übersteigt.

10. Kontrastmittel nach einem der vorangehenden Ansprüche, **dadurch gekennzeichnet, dass** mehrere vorzugsweise gleichartige Wechselwirkungen von vorzugsweise verschiedenen Atomen eines Retarder-Moleküls ausgehen und/oder an vorzugsweise verschiedenen Atomen eines oder mehrerer Tracer-Moleküle angreifen.

11. Kontrastmittel nach einem der vorangehenden Ansprüche, **dadurch gekennzeichnet, dass** der Tracer ein vorzugsweise wasserlösliches Biomolekül ist und/oder eine Struktur ausgewählt aus der Gruppe bestehend aus $^{13}C$-Atom, $^{15}N$-Atom, $^{29}Si$-Atom, $^{31}P$-Atom, $^{13}C$-Carbonylgruppe, $^{15}N$-Pyridinring aufweist.

12. Kontrastmittel nach einem der vorangehenden Ansprüche, **dadurch gekennzeichnet, dass** es sich bei dem Tracer und/oder dem Retarder um einen Stoff ausgewählt aus der Gruppe bestehend aus Nicotinamid, Nicotinsäure und Succinate handelt, insbesondere wobei der Tracer stofflich dem Retarder entspricht.

13. Kontrastmittel nach einem der vorangehenden Ansprüche, **dadurch gekennzeichnet, dass** es sich bei dem Retarder um einen Stoff ausgewählt aus der Gruppe bestehend aus Harnstoff, Thioharnstoff, Sulfaten, Cystein, Cystin, Amiden, Glycerinen, Dendrimeren von vorzugsweise einer zweiten Generation, Triphenylboran, Lewis-Säuren, insbesondere mit Bor als Lewis-saurem Zentrum, Metallen und Wasserstoffbrückendonoren handelt.

14. Kontrastmittel nach einem der vorangehenden Ansprüche, **dadurch gekennzeichnet, dass** es sich bei dem Lösungsmittel um Wasser handelt und Tracer und Retarder ausgewählt sind aus folgenden Tracer/Retarder-Paaren:

Nicotinamid/Harnstoff, Nicotinamid/Nicotinamid,
Pyridin/Nicotinamid, Pyridin/Harnstoff,
Pyrimidin/Nicotinamid, Pyrimidin/Harnstoff oder
Metronidazol/Harnstoff, Thioharnstoff/Pyridin,
Glucose/Pyridin, Ascorbinsäure/Pyridin, Milchsäure/Pyridin,
Pyridin/Triphenylboran.

15. Kontrastmittel nach einem der vorangehenden Ansprüche, **dadurch gekennzeichnet, dass** zumindest der Tracer hyperpolarisiert ist, insbesondere mittels DNP, PHIP und/oder SABRE, und/oder dass eine Konzentration des Retarders und/oder seiner Bindungsstellen größer als eine Konzentration des Tracers und/oder seiner Bindungsstellen ist und/oder dass eine Konzentration des Retarders größer als 100 mM ist.

16. Verfahren zur Herstellung eines hyperpolarisierten Kontrastmittel für die kernspinbasierte Bildgebung, **dadurch gekennzeichnet, dass** es sich bei dem hyperpolarisierten Kontrastmittel um ein Kontrastmittel nach einem der vorangehenden Ansprüche zumindest mit hyperpolarisiertem Tracer handelt, wobei die Hyperpolarisation mittels DNP, PHIP und/oder SABRE erfolgt und wobei der Retarder vor, während und/oder nach der Hyperpolarisation zugegeben wird.

17. Retarder zur Verwendung in einem Kontrastmittel für die kernspinbasierte Bildgebung, insbesondere einem Kontrastmittel nach einem der Ansprüche 1 bis 15 und/oder hergestellt in einem Verfahren nach Anspruch 16, zur bildgebenden Darstellung eines Stoffwechselprozesses, wobei der Retarder mit dem oder einem Tracer des Kontrastmittels wechselwirkt, wodurch eine T1-Relaxationszeit des Tracers verlängert wird.

18. Retarder nach dem vorangehenden Anspruch zur Anwendung in einem diagnostischen Verfahren in vivo und/oder in vitro, insbesondere zur Detektion von Erkrankungen, Überwachung von Krankheitsverläufen und/oder des Therapieansprechens, insbesondere bei Tumor-Erkrankungen und/oder metabolischen Erkrankungen.

19. Kontrastmittel nach einem der Ansprüche 1 bis 15 und/oder hergestellt in einem Verfahren nach Anspruch 16 zur Anwendung in einem diagnostischen Verfahren in vivo und/oder in vitro, insbesondere zur Detektion von Erkrankungen, Überwachung von Krankheitsverläufen und/oder des Therapieansprechens, insbesondere bei Tumor-Erkrankungen und/oder metabolischen Erkrankungen.

20. Verwendung eines Retarders nach einem der Ansprüche 17 oder 18 in einem vorzugsweise hyperpolarisierbaren Kontrastmittel für die kernspinbasierte Bildgebung zur Verlängerung der TI-Relaxationszeit des Tracers.

**Fig. 1**

Fig. 2

Europäisches
Patentamt

European
Patent Office

Office européen
des brevets

## EUROPÄISCHER RECHERCHENBERICHT

Nummer der Anmeldung

EP 24 18 5510

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | PETERS JOSH P. ET AL: "Nitrogen-15 dynamic nuclear polarization of nicotinamide derivatives in biocompatible solutions", SCIENCE ADVANCES, Bd. 9, Nr. 34, 23. August 2023 (2023-08-23), XP093231763, US ISSN: 2375-2548, DOI: 10.1126/sciadv.add3643 * das ganze Dokument * * Seite 3, linke Spalte, Absatz 5 * * Seite 11, rechte Spalte, Zeile 2 * ----- | 1-20 | INV. A61K49/10 A61K49/20 G01R33/28 |

RECHERCHIERTE
SACHGEBIETE (IPC)

A61K
G01R

Der vorliegende Recherchenbericht wurde für alle Patentansprüche erstellt

| Recherchenort | Abschlußdatum der Recherche | Prüfer |
|---|---|---|
| Den Haag | 12. Dezember 2024 | Mooren, Nicolai |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes
   Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- *CHEMICAL ABSTRACTS*, 98-92-0
- *CHEMICAL ABSTRACTS*, 39466-62-1
- *CHEMICAL ABSTRACTS*, 97-00-7
- *CHEMICAL ABSTRACTS*, 9002-07-7
- *CHEMICAL ABSTRACTS*, 57-13-6
- *CHEMICAL ABSTRACTS*, 1310-73-2